# EUROPEAN PATENT APPLICATION

(11) **EP 1 914 678 A1**
(43) Date of publication of application: **23.04.2008**
(21) Application number: 06782306.2
(22) Date of filing: 28.07.2006
(51) Int. Cl.: G06Q 50/00, A23L 1/29, A61K 8/19, A61K 8/30, A61K 8/96

(54) **METHOD OF PROVIDING PREPARATION FOR TOPICAL APPLICATION AND SUPPLEMENT MEETING CLIENT'S NEEDS**

(30) Priority: 28.07.2005 JP 2005219669
(71) Applicant: Ajinomoto Co., Inc., Tokyo 104-8315 (JP)
(72) Inventor: TAKINO, Yoshinobu, c/o AJINOMOTO CO., INC., Kawasaki-shi, Kanagawa 210-8681 (JP); SAGAWA, Koichiro, c/o AJINOMOTO CO., INC., Kawasaki-shi, Kanagawa 210-8681 (JP); AMINO, Yusuke, c/o AJINOMOTO CO., INC., Kawasaki-shi, Kanagawa 210-8681 (JP)
(74) Representative: Johnson, Richard Alan
(86) International application number: PCT/JP2006/315446
(87) International publication number: WO 2007/013690

(57) **Abstract**

The present invention provides a method of efficiently providing, to a client, a preparation for topical application and a supplement suitable for the client, which includes inputting a part desired by the client to be improved and an improvement effect desired for the part, determining, based on the information and by a computer, a composition of nutritional components suitable for the improvement effect desired for the part to be improved, determining a preparation for topical application for delivering the selected nutritional components to the part to be improved, determining a supplement containing the selected composition of nutritional components by referring to the database, in which compositions of nutritional components of supplements are registered in advance, and outputting the information of the selected supplement and preparation for topical application.

SEQUENCE LISTING

<110> AJINOMOTO CO., INC.

<120> METHOD OF PROVIDING PREPARATION FOR TOPICAL APPLICATION AND SUPPLEMENT MEETING CLIENT'S NEEDS

<130> 09940

<150> JP 2005-219669
<151> 2005-07-28

<160> 3

<170> PatentIn version 3.3

<210> 1
<211> 6
<212> PRT
<213> Artificial

<220>
<223> Peptide for use in anti-wrinkle by a relaxation of the muscles to
      control exocytosis at neuron

<400> 1

Glu Glu Met Gln Arg Arg
1               5


<210> 2
<211> 4
<212> PRT
<213> Homo sapiens

<400> 2

Ala Glu Asp Gly
1


<210> 3
<211> 4
<212> PRT
<213> Glycine max

<400> 3

Met Ile Thr Leu
1

## Description

### Technical Field

The present invention relates to a method of efficiently providing a preparation for topical application and a supplement suitable for a client, a system therefor, a program used therefor, as well as a combination product of a preparation for topical application and a supplement.

### Background Art

Conventionally, in the field of cosmetics, when beautiful skin effects such as whitening, moisturizing, improving skin fitness and the like (hereinafter to be also collectively referred to simply as "beautiful skin effect") are desired to be achieved in the skin with cosmetics, an external application form of the cosmetics is generally employed. From the aspects of "becoming beautiful from the inside" in recent years, various supplements aiming at beautiful skin effect, which contain vitamins, collagen and the like, are placed on the market. In addition, kits comprising cosmetics aiming at the same effect as provided by such supplements are also disclosed (JP-A-2004-75558, JP-A-2004-107238, JP-A-2004-107242, JP-A-2004-107243 and the like). When such supplement is taken, the part where the beautiful skin effect is generally expected is mostly the face. However, when taken orally, various nutritional components contained in the supplement do not act on the face from the inside because they are delivered throughout the body in the bloodstream. For example, if cysteine (a known whitening component) should directly act on melanin from the inside of the face skin and exhibit a whitening effect, it will also act simultaneously on the scalp melanin and the black hair will be discolored. However, since the discoloration of the black hair does not occur, the whitening effect of cysteine is considered to be afforded via the improvement of visceral function and the like.

On the other hand, in the field of pharmaceutical products, an embodiment is known in which a topical administration of a blood circulation improving agent is combined with a systemic administration of a pharmaceutical agent associated with many side effects such as anticancer agents and the like, in an attempt to afford a topical effect of the pharmaceutical agent (US Patent No. 5620416). However, this is a special form of treatment based on doctor's prescription and instructions, which is not easily available to the public and is not prevalent much among the public. In addition, it is not known to use an appropriate combination of material outside pharmaceutical products.

### Disclosure of the Invention

Accordingly, an effective method of providing a preparation for topical application, which can be used conveniently by the public without doctor's prescription and which effectively affords a topical improvement effect, is desired.

In view of the above-mentioned background, the present invention mainly aims at providing a method of effectively providing, to a client, a preparation for topical application (cosmetic and quasi drug) effective for increasing concentrations of various supplements at a topical part where an improvement is desired.

To be specific, the present invention provides a method of efficiently providing a supplement and a preparation for topical application, which are suitable for a client, a system therefor and a program used therefor, and furthermore, a supplement to be used in combination with a preparation for topical application, which is suitable for effectively achieving a topical effect desired by the client, and a preparation for topical application, which is to be used in combination with the supplement, and a combination product thereof, in order to effectively achieve the topical effect desired by the client, thereby meeting the request of the client.

The gist of the present invention is as follows.
[1] A method of providing information of a supplement and a preparation for topical application, which are suitable for a client, which comprises
   a step of inputting a part desired by the client to be improved and an improvement effect desired for said part into an input tool,
   a step for a computer to determine a composition of nutritional components suitable for the improvement effect desired for the part desired by the client to be improved, by referring to the information stored in a database, in which information of the correlation between improvement effects desired for parts to be improved and compositions of nutritional components is registered in advance, and extracting, from the information contained in the database, information of the composition of the nutritional components suitable for the improvement effect desired for the part to be improved,
   a step for the computer to determine a preparation for topical application, which is suitable for delivering the determined nutritional components to the part to be improved, by referring to the information stored in a database, in which information of the correlation between parts of the body and preparations for topical application suitable for delivering nutritional components to the parts is registered in advance, and extracting, from the information contained in the database, a preparation for topical application suitable for the part desired by the client to be improved,
   a step for the computer to determine a supplement containing the composition of the determined nutritional components, by referring to a database, in which supplements and compositions of nutritional components thereof are registered in advance, and extracting, from the information in the database, information of a supplement corresponding to the composition of the determined nutritional components, and
   a step of outputting the information of the supplement and the preparation for topical application as determined by the computer to an output tool.
[2] The method of the above-mentioned [1], further comprising a step of outputting a method of using the determined supplement and the preparation for topical application in combination and/or the effect expected by the combined use to the output tool.
[3] The method of the above-mentioned [1] or [2], wherein the part desired by the client to be improved is the skin.
[4] The method of any of the above-mentioned [1] to [3], wherein the improvement effect desired for the part to be improved is at least one kind selected from the group consisting of improvement and/or prophylaxis of skin wrinkles, improvement and/or prophylaxis of skin pigmentation, promotion of hair growth in the scalp and prevention of gray hair.
[5] The method of any of the above-mentioned [1] to [4], wherein the composition of the determined nutritional components comprises at least one kind selected from the group consisting of vitamin, amino acid, peptide, protein, mineral, dietary fiber, fatty acid, carbohydrate, nucleic acid, organic acid, fungi, extract from plants and animals, and lipid.
[6] The method of any of the above-mentioned [1] to [5], wherein the determined preparation for topical application is a blood circulation improving agent comprising a blood circulation promoting component.
[7] The method of the above-mentioned [6], wherein the blood circulation promoting component comprises at least one kind of active ingredient selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.
[8] The method of any of the above-mentioned [1] to [7], wherein the determined supplement comprises at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, the determined preparation for topical application comprises a blood circulation promoting component, and the improvement effect desired for the part to be improved is an improvement and/or prophylaxis of skin pigmentation.
[9] The method of any of the above-mentioned [1] to [7], wherein the determined supplement comprises at least one kind selected from the group consisting of mineral, vitamin, amino acid, carbohydrates, lipid, inositol, sulfur, nucleic acid, collagen, sesaminol and citric acid, the preparation for topical application comprises a blood circulation promoting component, and the improvement effect desired for the part to be improved is promotion of hair growth in the scalp.
[10] A method of providing information of a preparation for topical application suitable for a supplement selected by a client, which comprises
   a step of inputting information of the supplement into an input tool,
   a step for a computer to determine a preparation for topical application suitable for delivering nutritional components contained in the supplement to a part to be improved, by referring to the information stored in a database, in which information of the correlation between supplements and preparations for topical application suitable for delivering nutritional components contained in the supplements to parts to be improved is registered in advance, and extracting, from the information contained in the database, the information of the preparation for topical application suitable for delivering the nutritional components contained in the input supplement to the part to be improved, and
   a step of outputting the information of the preparation for topical application as determined by the computer into an output tool.
[11] The method of the above-mentioned [10], further comprising a step of outputting a method of using the supplement and the determined preparation for topical application in combination and/or the effect expected by the combined use to the output tool.
[12] The method of the above-mentioned [10] or [11], wherein the determined preparation for topical application is a blood circulation improving agent containing a blood circulation promoting component.
[13] The method of the above-mentioned [12], wherein the blood circulation promoting component comprises at least one kind of active ingredient selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.
[14] A method of providing information of a supplement suitable for a preparation for topical application selected by a client, which comprises
   a step of inputting information of the preparation for topical application into an input tool,
   a step for a computer to select a supplement suitable for delivering the preparation for topical application to a part to be improved, by referring to the information stored in a database, in which information of the correlation between preparations for topical application and supplements suitable for delivering preparations for topical application to parts to be applied is registered in advance, and extracting, from the information contained in the database, information of a supplement suitable for delivering the preparation for topical application to the part to be applied, and
   a step of outputting the information of the supplement as determined by the computer into an output tool.
[15] The method of the above-mentioned [14], further comprising a step of outputting a method of using the supplement and the determined preparation for topical application in combination and/or an effect expected by the combined use to the output tool.
[16] The method of the above-mentioned [14] or [15], wherein the determined preparation for topical application is a blood circulation improving agent containing a blood circulation promoting component.
[17] The method of the above-mentioned [16], wherein the blood circulation promoting component comprises at least one kind of active ingredient selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.
[18] A method for the prophylaxis or improvement of skin pigmentation, which comprises orally administering a supplement comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide before or after transdermal application of a preparation for topical application comprising at least one kind of blood circulation promoting component selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.
[19] A kit for the prophylaxis or improvement of skin pigmentation, consisting of a preparation for topical application comprising at least one kind of blood circulation promoting component selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract, and a supplement comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, which is used to be orally administered to a test subject to whom the preparation for topical application is applied.
[20] An agent for the prophylaxis or improvement of skin pigmentation, comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, which is used for the prophylaxis or improvement of skin pigmentation of a test subject to whom a preparation for topical application comprising at least one kind of blood circulation promoting component selected from the group consisting of capsinoids, capsinoid derivative, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract is applied to a part where prophylaxis or improvement of the skin pigmentation is desired.

### Brief Description of the Drawings

Fig. 1 is a flow chart explaining each step performed in the method and system of the present invention.
Fig. 2 is a flow chart explaining each step performed in the method and system of the present invention.
Fig. 3 is a flow chart explaining each step performed in the method and system of the present invention.
Fig. 4 is one example of a system for practicing the method of the present invention.
Fig. 5 is one example of a system via a network for practicing the method of the present invention.

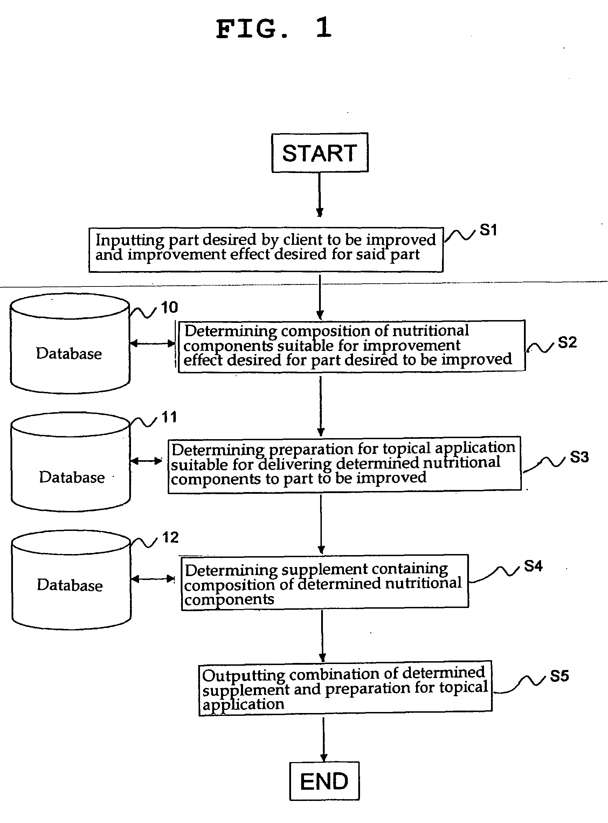

   In Figs. 1 to 5, 1 is an input tool or output tool, 2 is a central arithmetic device (computer), 3 is a management server, 10 is a database containing information of the correlation between an improvement effect desired for a part to be improved and a composition of nutritional components, 11 is a database containing information of the correlation between parts of the body and preparations for topical application suitable for the parts, 12 is a database registering supplements and compositions of nutritional components thereof, 20 is a database registering the information of the correlation between supplements and preparations for topical application, 30 is a database registering information of the correlation between preparations for topical application and supplements, and 100 is a network.

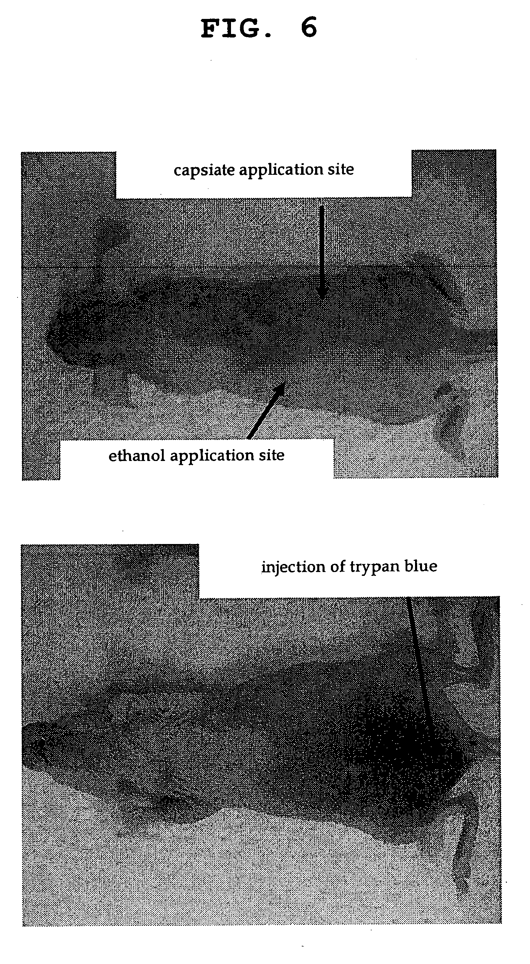

Fig. 6 is a photograph showing the results of Example 1.

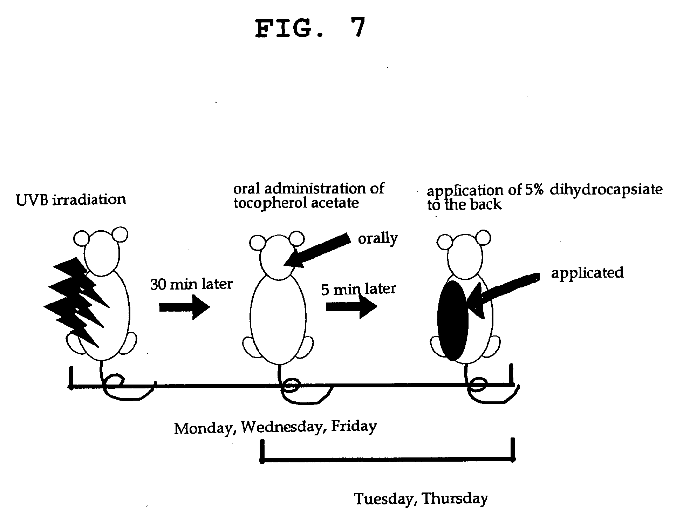

Fig. 7 is a flow chart showing the treatments performed on hairless mouse in Example 2.

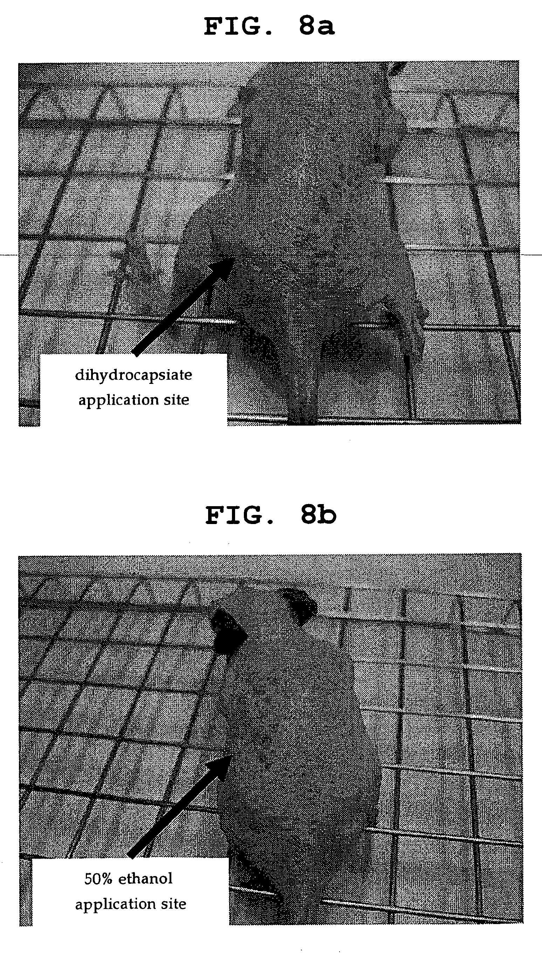

Figs. 8a - c are photographs showing a suppressive effect by a combined use of oral administration of tocopherol acetate and transdermal application of dihydrocapsiate against pigmentation induced by ultraviolet beam (UVB) (Example 2).

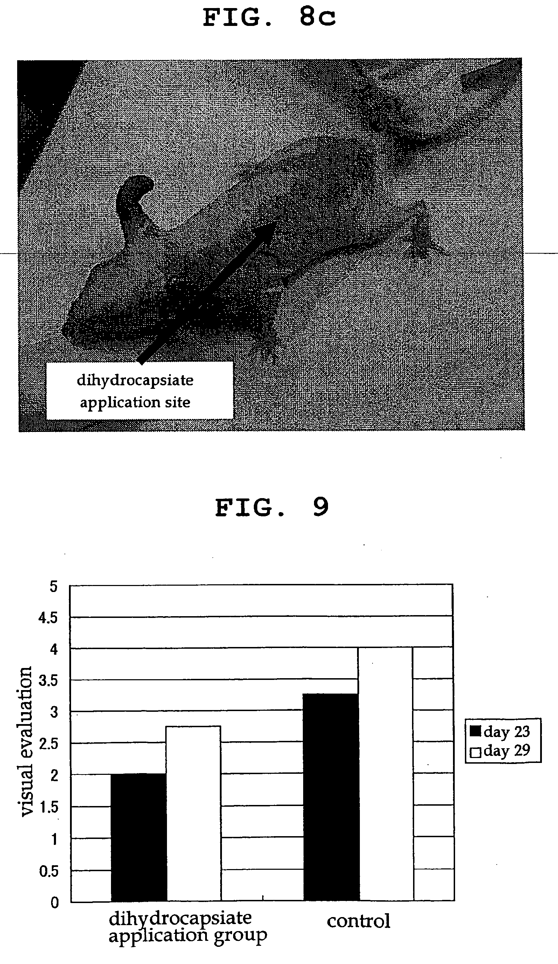

Fig. 9 is a graph showing a suppressive effect by a combined use of oral administration of cystine and transdermal application of dihydrocapsiate against pigmentation induced by ultraviolet beam (UVB) (Example 3).

### Detailed Description of the Invention

In the present invention, a preparation for topical application preferably means a preparation for topical application classified into cosmetic agents and quasi drugs that do not require doctor's prescription.

The part desired by the client to be improved is not particularly limited as long as it is a part of the body where the client desires an improvement. Examples thereof include parts such as skin, scalp, mucous membrane of the nasal cavity, mucous membrane of the mouth cavity, eyeball, supporting tissue, muscle, nerve, blood vessel and the like. Of these, the part to be improved in the present invention is preferably a part the client can treat by him/herself, and the skin (including scalp) is particularly preferable as the part to be improved.

The "improvement effect desired for the part to be improved" is not particularly limited as long as it is the effect the client desires to be achieved in the part the client desires. It may be an effect improving existing symptoms, or preventing an undesired symptom. Examples of such effect include prophylaxis or improvement of skin wrinkles, prophylaxis or improvement of skin pigmentation, promotion of hair growth in the scalp, prophylaxis or improvement of gray hair, prophylaxis or improvement of skin sagging, prophylaxis or improvement of dull skin, prophylaxis or improvement of skin pore expansion, prophylaxis or improvement of reduced skin barrier ability, prophylaxis or improvement of reduced skin moisturizing effect, normalization of skin turnover, suppression of skin inflammation, prophylaxis or improvement of blemish skin, improvement of skin itchiness, prophylaxis or improvement of skin acne, promotion of skin slimming and the like, with preference given to improvement of skin wrinkles, prophylaxis or improvement of skin pigmentation, promotion of hair growth in the scalp and prevention of gray hair. The improvement effect may not be only one kind but two or more kinds of improvement effects may be simultaneously desired as appropriate.

Examples of the component to be contained in the composition of nutritional components in a supplement include, but are not particularly limited to, those contained in the supplements and products currently marketed, components expected for application to supplements from pharmaceutical products, and the like. Specific examples include vitamin, amino acid, peptide, protein, mineral, dietary fiber, fatty acid, carbohydrates, nucleic acid, organic acid, fungi, an extracts from plants and animals, lipid and the like, with preference given to vitamin, amino acid, organic acid, nucleic acid, fatty acid and carbohydrate. Plural components may also be contained simultaneously.

Examples of the vitamin include liposoluble vitamins such as β-carotene, vitamin D, vitamin E, vitamin E derivative, vitamin K, coenzyme Q10 and the like, aqueous vitamins such as vitamin B1, vitamin B2, vitamin B6, vitamin B12, niacin, pantothenic acid, folic acid, vitamin C, etc. and the like.

Examples of the amino acid include glycine, alanine, valine, leucine, isoleucine, serine, threonine, cysteine, tyrosine, methionine, asparagine, aspartic acid, glutamic acid, glutamine, arginine, lysin, histidine, phenylalanine, tryptophan, proline, homoserine, ornithine, citrulline, carnitine, β-alanine and the like.

Examples of the peptide include various dipeptides (Ala-Gln, γ-Glu-Cys, γ-Glu-Lys, Leu-Phe, N-Alanyl-3-methyl-L-His, β-Ala-His(Carnosine), β-Ala-1-MeHis (Anserine), Arg-Gly, Arg-Ala, Arg-Leu, Arg-Asp, Arg-Glu, Arg-Lys, Cys-Gly, Cys-Arg, Cys-Cys, Cys-His, Cys-Pro, Ala-Cys, α-Glu-Cys, Lys-Cys, Arg-Cys, Gly-Tyr, Ala-Tyr, Arg-Tyr, Cys-Tyr, Gly-Leu, Leu-Leu, His-Leu, Leu-Gly, Leu-Ala, Leu-Arg, Leu-His, Leu-Ser), oligopeptide (γ-Glu-Cys-Gly (Glutathione), Glu-Glu-Met-Gln-Arg-Arg (SEQ ID NO: 1), Ala-Glu-Asp-Gly (Epitalon;SEQ ID NO: 2), Gly-L-His-L-Lysine:copper, Met-Ile-Thr-Leu (SEQ ID NO: 3)), various amides and esters of these peptides, soybean peptide, lactoprotein decomposed product (β-casein, α-lactoalbumin, β-lactoglobulin, casein, lactoferrin, whey protein) and the like.

Any protein can be used without any particular limitation, as long as it is a protein constituted with various amino acids. Examples thereof include animal proteins such as various lactoproteins, collagen, elastin and the like, plant proteins such as silk protein, soybean protein, etc. and the like.

Examples of the mineral include calcium, phosphorus, iodine, selenium, magnesium, zinc, iron, potassium and the like.

Examples of the dietary fiber include plant cell wall components such as cellulose, lignin, hemicellulose and the like, soluble saccharides not digested with amylase, indigestible components derived from animal food and the like.

Examples of the fatty acid include various saturated fatty acids, unsaturated fatty acids such as linoleic acid, γ-linolenic acid, DHA (docosahexaenoic acid), EPA (eicosapentaenoic acid), etc. and the like.

Examples of the carbohydrate include monosaccharides such as glucose, fructose and the like, oligosaccharides such as fructooligosaccharide (starting material: sucrose), soybean oligosaccharide (starting material: soybean), lactosucrose (starting material: sucrose and sugar), raffinose (starting material: beet molasses), glactooligosaccharide (starting material: lactose), xylo-oligosaccharide (starting material: corn), etc. and the like.

Examples of the nucleic acid include DNA, RNA and the like of various chain lengths.

Examples of the organic acid include citric acid, malic acid and the like.

Examples of the fungi include bacteria such as lactic acid bacteria, bifidobacteria and the like, which grow on lactose, glucose and the like as nutrients, and produce lactic acid, etc. and the like.

Examples of the extracts from plants and animals include polyphenol, anthocyanin, isoflavone, catechin, curcumin, flavonoid, sesaminol, soybean saponin, ellagic acid, ginkgolides, allicin, glutathione, cystatin, chlorophyll, carotenoid, α-carotene, lutein, lycopene, fucoxanthine, tannic acid, theaflavin, chlorella and the like.

Examples of the lipid include, in addition to the aforementioned fatty acid, acylglycerol (monoacylglycerol, diacylglycerol, triacylglycerol), squalene, wax, cholesterol and fatty acid ester thereof, glycerolphospholipid (lecithin and the like), sphingophospholipid (sphingomyelin and the like), various fatty acid esters, phytosterol and the like.

The supplement here refers to quasi drug, food, nutraceutical food, health food, functional health food and the like other than pharmaceutical products requiring doctor's prescription. Examples thereof include those containing vitamin, amino acid, organic acid and the like as components, such as those mentioned above. The supplements may be blended with a food as long as they are in a drinkable form. They may be in any form of powder, tablet, capsule, drink and the like, and may appropriately contain an excipient generally used for preparations.

The components that can be contained in supplements for the object meeting the improvement effect desired by a client for a part to be improved are recited in the following. However, the supplements to which the present invention can be applied are not limited to them.
(1) Whitening supplements
   Examples of the component a supplement effective for whitening (including prophylaxis or improvement of skin pigmentation) may contain (hereinafter to be also referred to as "whitening supplement") include acylated or esterified cysteine such as cysteine, cysteine sulfinic acid, glutathione (L-Glu-L-Cys-Gly), γ-Glu-Cys, N-acetylcysteine and the like, cysteine derivative containing homocysteine, cystathionine, cysteine precursor (serine, methionine, etc.) and the like, cystine derivative containing cystine, acylated or esterified cystine (N,N-diacetyl-L-cystine dimethyl ester N,N-diacetyl-L-cystine diethyl ester, N,N-diacetyl-L-cystine dipropyl ester, N,N-diacetyl-L-cystine dibutyl ester, N,N-diacetyl-L-cystine dihexyl ester, N,N-diacetyl-L-cystine dioctyl ester, etc.) and the like, vitamin C, polyphenol, tannic acid, vitamin E, vitamin E derivative containing acetic acid derivative such as tocopherol acetate, etc. and the like, catechin, niacin, hydroxytyrosol, unsaturated fatty acid (palmitoleic acid, oleic acid, elaidic acid, vaccenic acid, erucic acid, α-linolenic acid, arachidonic acid, DHA (docosahexaenoic acid), EPA (eicosapentaenoic acid), docosapentanoic acid, linoleic acid, γ-linolenic acid, etc.), vitamin P (flavones, rutin, hesperidin, citrin), vitamin Q (ubiquinone), glutathione, isoflavone, guava, selenium, zinc oxide and the like. Of these, cysteine, vitamin C, cystine, glutathione (L-Glu-L-Cys-Gly), γ-Glu-Cys, vitamin P (rose hip, etc.) are particularly preferable. The contents of these components can be appropriately determined using the maximum acceptable daily intake of the selected component as an index.
(2) Moisturizing supplements
   Examples of the supplement effective for moisturization (hereinafter to be also referred to as "moisturizing supplement") include vitamin B2, vitamin B6, vitamin E, vitamin A, vitamin C, chondroitin sulfate, squalene (shark liver extract, olive oil, cottonseed oil, avocado oil, etc.), collagen, zinc, chamomile, coenzyme Q10, wheat germ, adlay, hyaluronic acid, isoflavone and the like. The contents of these components can be appropriately determined using the maximum acceptable daily intake of the selected component as an index.
(3) Hair growth supplements
   Examples of the supplement effective for hair growth (hereinafter to be also referred to as "hair growth supplement") include amino acid such as histidine, glutamine and the like, seaweed extract (alginic acid, agar, carageenan, fucoidan, etc.), various minerals containing zinc, sulfur, iodine, iron, calcium, copper, phosphorus, zinc and the like, vitamins such as vitamin A, vitamin B2, vitamin B6, pantothenic acid (vitamin B5), vitamin C, vitamin E, vitamin F (unsaturated fatty acid), vitamin B15 (pangamic acid), vitamin Q (ubiquinone), biotin (vitamin H), folic acid, niacin (nicotinic acid), coenzyme Q10 and the like, amino acid such as histidine, glutamine and the like, carbohydrates, lipid, inositol, sulfur, nucleic acid (adenosine and phosphate ester thereof, etc.), collagen, sesaminol, citric acid and the like. Of these, histidine, glutamine, adenosine and phosphate ester thereof are particularly preferable. The contents of these components can be appropriately determined using the maximum acceptable daily intake of the selected component as an index.
(4) Antiaging supplements
   Examples of the supplement effective for antiaging (particularly, antiaging of skin) include amino acid such as proline, alanine, glutamine, cystine, teanin and the like, antiaging component such as carotenoid (β-carotene, lutein, lycopene), vitamin B1 (thiamine), vitamin B2 (riboflavin), niacin (nicotinic acid), vitamin A, vitamin E, vitamin B15 (pangamic acid), paraaminobenzoic acid, lipoic acid, vitamin C, vitamin Q (ubiquinone), phosphorus, sulfur, vitamin P (flavones, rutin, hesperidin, citrin), vitamin Q (ubiquinone), copper, manganese, selenium, silicon, collagen, polyphenol (anthocyanin, catechin, cacaomas polyphenol, quercetin, isoflavone, quercetin, chlorogenic acid), sesaminol (sesame), soybean saponin, ellagic acid, glutathione, chlorophyll, curcumin, young barley grass extract (SOD; superoxide· dismutase), nucleic acid (DNA, RNA, etc.), guava, wheat germ, beer yeast (glutathione, etc.), prune, rose hip (vitamin P, etc.) and the like, collagen, soybean peptide and the like. Of these, vitamins and amino acids are particularly preferable. The contents of these components can be appropriately determined using the maximum acceptable daily intake of the selected component as an index.
(5) Anti-gray hair supplements

Examples of the supplement effective for anti-gray hair (hereinafter to be also referred to as "anti-gray hair supplement") include biotin, tyrosine, paraaminobenzoic acid, copper, collagen, sesaminol and the like. Of these, tyrosine and paraaminobenzoic acid are particularly preferable. The contents of these components can be appropriately determined using the maximum acceptable daily intake of the selected component as an index.

As the preparation for topical application, any can be used without particularly limitation as long as it can deliver the aforementioned components of the supplements throughout the topical application site, preferably maintain a high topical concentration as compared to non-administration. The preparation for topical application is preferably a blood circulation improving agent containing a blood circulation promoting component. The blood circulation promoting component is not particularly limited as long as it increases blood flow of a desired application site by application to the site. Various components such as capsinoids and various derivatives thereof, pyrrolidone carboxylic acid (PCA) and a salt thereof (zinc salt, fatty acid salt, sodium salt, potassium salt, silver salt, copper salt, arginine salt, etc.), esters of pyrrolidone carboxylic acid (methyl ester, ethyl ester, propyl ester, butyl ester, pentyl ester; hexyl ester, heptyl ester, octyl ester, nonyl ester, decyl ester, undecyl ester, dodecyl ester, tridecyl ester, tetradecyl ester, pentadecyl ester, hexadecyl ester, heptadecyl ester, octadecyl ester, nonadecyl ester, eicosyl ester, isopropyl ester, isobutyl ester, s-butyl ester, t-butyl ester, isopentyl ester, t-pentyl ester, neopentyl ester, isohexyl ester, cyclohexyl ester, benzyl ester, etc.), arginine and a salt thereof (hydrochloride, acetate, sulfate, fatty acid salt, etc.), arginine oligomer (octamer-dodecamer), nicotinic acid, niacin, capsaicin, vitamin E (α-tocopherol, etc.), Swertia japonica extract (Swertinogen), cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract, Ginkgo extract and the like can be used, which are appropriately selected according to the application site and object of a preparation for topical application, and one or more kinds of which may be contained in a preparation for topical application. Here, capsinoids refer to fatty acid ester of vanillyl alcohol, and representative substances thereof include capsiate, dihydrocapsiate, and nordihydrocapsiate, which have been confirmed as components contained in capsicums. The present invention also encompasses fatty acid esters of various straight chain or branched chain fatty acid and vanillyl alcohol, which have a fatty acid chain length similar to that of capsiate and nordihydrocapsiate, such as vanillyldecanoate, vanillylnonanoate, vanillyloctanoate and the like. Capsiate, dihydrocapsiate and nordihydrocapsiate can be respectively represented by the following chemical formulas. Capsinoids are extremely low-stimulant unlike capsaicin, due to their structural characteristics. In addition, since the effect is temporary (about 15 min to 5 hr after application to the skin), they are preferable for providing a specific topical effect only for a given period of time.

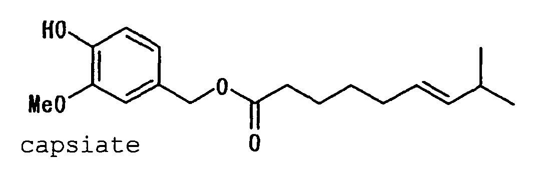

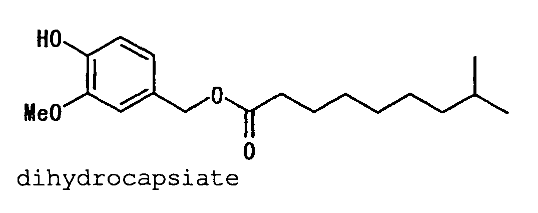

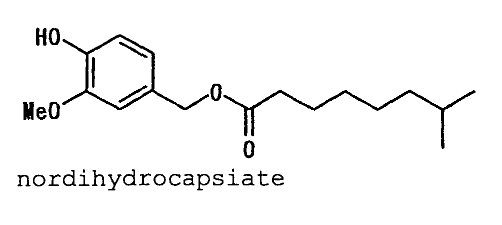

In addition, these preparations for topical application can appropriately contain other components to provide an effect expected to be afforded by a combined use. While the contents of such components vary depending on the kind of the component, they only need to be contained in an amount affording a desired topical effect. For example, they can be contained in a proportion of about 0.01 - 10 wt% in a preparation for topical application.

Examples of the preparation for topical application include the following preparations. However, the preparation for topical application, to which the present invention can be applied, is not limited to them.
(1) Preparation for topical application to the skin
   When a preparation for topical application is used for the skin, to which normal cosmetics are applied, such as face, neck, hand and the like (hereinafter to be also referred to as "preparation for topical application to the skin"), the preparation may further contain a component contained in normal cosmetics, as long as the components of the aforementioned supplements can be delivered throughout the topical application site. In addition, when a preparation for topical application contains a blood circulation promoting component, the preparation only needs to be in a use mode permitting the blood circulation promoting component to exhibit efficacy. Furthermore, when a preparation for topical application primarily seeks to achieve a whitening effect (hereinafter to be also referred to as "whitening preparation for topical application"), the preparation may contain, along with a blood circulation promoter, a whitening component contained in normal whitening cosmetics.
   Examples of the component contained in normal cosmetics include oil (olive oil, Camellia japonica oil, macadamia nuts oil, castor oil, etc.), waxes (Carnauba wax, candelilla wax, jojoba oil, beeswax, lanolin, etc.), hydrocarbon (liquid paraffin, paraffin, vaseline, ceresin, microcrystalline wax, squalane, etc.), higher fatty acid (lauric acid, myristic acid, palmitic acid, stearic acid, isostearic acid, etc.), higher alcohol (cetyl alcohol, stearyl alcohol, isostearyl alcohol, 2-octyldodecanol, etc.), esters (isopropyl myristate, 2-octyldodecyl myristate, cetyl 2-ethylhexanoate, diisostearyl malate, etc.), silicone oil (methylpolysiloxane, methylphenylpolysiloxane, etc.), anion surfactant (higher fatty acid soap, alkylsulfate, polyoxyethylenealkyl sulfate, acyl N-methyl taurinate, alkyletherphosphate, N-acylamino acid salt (N-acylglutamic acid salt, N-acyl-N-methyl-β-alanine salt, N-acyl sarcosinate, etc.) and the like), cationic surfactant (ammonium alkyltrimethyl chloride, ammonium dialkyldimethyl chloride, benzalkonium chloride, etc.), amphoteric surfactant (betaine alkyldimethylaminoacetate, betaine alkylamidedimethylaminoacetate, 2-alkyl-N-carboxy-N-hydroxyimidazolium, etc.), nonionic surfactant (fatty acid monoglyceride ester type (fatty acid is lauric acid, myristic acid, palmitic acid, oleic acid, linoleic acid, ricinoleic acid, isopalmitic acid, isostearic acid, etc.), propylene glycol fatty acid ester type (fatty acid is lauric acid, myristic acid, palmitic acid, oleic acid, linoleic acid, ricinoleic acid, isopalmitic acid, isostearic acid, etc.), sucrose fatty acid ester type (fatty acid is lauric acid, myristic acid, palmitic acid, oleic acid, linoleic acid, ricinoleic acid, isopalmitic acid, isostearic acid, etc.), higher alcohol oxidization ethylene condensation type (alcohol is lauryl alcohol, cetyl alcohol, stearyl alcohol, oleyl alcohol, lanolin alcohol, isostearyl alcohol, etc.), polyethylene glycol fatty acid ester type (fatty acid is lauric acid, myristic acid, palmitic acid, oleic acid, linoleic acid, ricinoleic acid, isopalmitic acid, isostearic acid, etc.), polyethylene glycol sorbitan fatty acid ester type (fatty acid is lauric acid, myristic acid, palmitic acid, oleic acid, linoleic acid, ricinoleic acid, isopalmitic acid, isostearic acid, etc.), fatty acid alkylolamide type (fatty acid is lauric acid, myristic acid, palmitic acid, oleic acid, linoleic acid, ricinoleic acid, isopalmitic acid, isostearic acid, etc., alkylolamine is monoethanol, diethanolamine, isopropanolamine, etc.) and the like), ethyleneoxide· propyleneoxide block copolymer, moisturizing agent (glycerol, propylene glycol, 1,3-butyleneglycol, polyethylene glycol, sorbitol, sodium lactate, sodium 2-pyrrolidon-5-carboxylate, sodium hyaluronate, etc.), natural polymer compound (gum arabic, gum tragacanth, guar gum, locust gum, gum karaya, Irish moss, pyrus cydonia seed, gelatin, shellac, rosin, casein, etc.), semisynthetic polymer compound (sodium carboxymethylcellulose, hydroxyethylcellulose, methylcellulose, ethylcellulose, sodium alginate, ester gum, nitrocellulose, hydroxypropylcellulose, crystalline cellulose, etc.), synthesis polymer compound (polyvinyl alcohol, polyvinylpyrrolidone, sodium polyacrylate, carboxyvinyl polymer, polyvinylmethyl ether, polyamide resin, silicone oil, etc.), UV absorber (2-hydroxy-4-methoxybenzophenone, 2-hydroxy-4-methoxybenzophenon-5-sulfonic acid, sodium 2-hydroxy-4-methoxybenzophenon-5-sulfonate, dihydroxydimethoxybenzophenone, sodium dihydroxydimethoxybenzophenone-sulfonate, 2,4-dihydroxybenzophenone, tetrahydroxyberizophenone, paraaminobenzoic acid, ethyl paraaminobenzoate, glyceryl paraaminobenzoate, amyl paradimethylaminobenzoate, octyl paradimethylaminobenzoate, ethyl paramethoxycinnamate, isopropyl paramethoxycinnamate, octyl paramethoxycinnamate, 2-ethoxyethyl paramethoxycinnamate, sodium paramethoxycinnamate, potassium paramethoxycinnamate, glyceryl paramethoxycinnamic acid mono-2-ethylhexanoate, octyl salicylate, phenyl salicylate, homomenthyl salicylate, dipropyleneglycol salicylate, ethyleneglycol salicylate, myristyl salicylate, methyl salicylate, urocanic acid, ethyl urocanate, 4-tert-butyl-4'-methoxybenzoylmethane (parsol A), 2-(2'-hydroxy-5'-methylphenyl)benzotriazole, methylanthranilate, etc.), antioxidant (tannic acid, tocopherol, BHT (dibutylhydroxytoluene), BHA (butylhydroxyanisole), gallic acid ester, NDGA (nordihydroguayaretic acid), etc.), antioxidation agent (phosphoric acid, citric acid, ascorbic acid, maleic acid, malonic acid, succinic acid, fumaric acid, cephalin, hexametaphosphate, phytic acid, EDTA (ethylenediamine tetraacetic acid), etc.); metal blocking agent (EDTA (ethylenediamine tetraacetic acid) sodium salt, phosphoric acid, citric acid, ascorbic acid, succinic acid, gluconic acid, sodium polyphosphate, sodium metaphosphate, etc.), azo dye (Yellow No. 5, Red No. 505, etc.), xanthene dye (Red No. 230, Red No. 223, Red No. 213, etc.), quinoline dye (Yellow No. 204, Yellow No. 203, etc.), triphenylmethane dye (Blue No. 1, etc.), anthraquinone dye (Green No. 201, Green No. 202, etc.), indigo dye (Blue No. 2, etc.), nitro dye (Yellow No. 403, etc.), pyrene dye (Green No. 204, etc.), nitroso dye (Green No. 401, etc.), lake (Red No. 201, Red No. 204, Red No. 206, Red No. 207, Red No. 208, Red No. 220, Yellow No. 5, Red No. 230, etc.), organic pigment (azo pigment (Red No. 228, etc.), indigo pigment (Red No. 226, etc.), phthalocyanine pigment (Blue No. 404, etc.) and the like), natural dye (β-carotene, carthamin, cochineal, etc.), body pigment (mica, talc, kaolin, etc.), coloring pigment (red oxide of iron, yellow oxide of iron, black oxide of iron, ultramarine blue pigment, etc.), white pigment (titanium dioxide, zinc oxide, etc.), pearl gloss pigment (dioxide coated titanated mica, etc.), polymer powder (polyethylene powder, polymethyl methacrylate, poly(ethylene terephthalate)· polymethylmethacrylate laminate powder, nylon powder, etc.), functional pigment (boron nitride, synthesis fluorine mica (synthesis mica), photochromic pigment, etc.), complex fine particles powder (hybrid·finepowder, etc.), flavor (limonene, β-caryophyllene, cis-3-hexenol, linalool, Farnesol, β-phenylethyl alcohol, citral, α-hexyl cinnamic aldehyde, β-ionone, 1-carbone, cyclopentadecanone, linalyl acetate, benzylbenzoate, γ-undecalactone, eugenol, rose oxide, indole, phenylacetoaldehydedimethylacetal, oranthiol, 2,6-nonadienal, geraniol, citronellol, terpineol, menthol, santalol, Bacdanol, bramanol, lyral, lirial, damascone , methyl ionone, irone, Iso E Super, acetylcedrene, muscone, benzylacetate, methyldihydro jasmonate, methyl jasmonate, jasmine lactone,cyclopentadecanorid, ethylene bracilate, garaksolid, ambroxan, etc.), anti-inflammatory agent (β-glycyrrhetinic acid, glycyrrhizic acid derivative, allantoin, azulene, s-aminocaproic acid, hydrocortisone, hydrocortisone acetate, prednisolone, etc.), astringent agent (zinc oxide, zinc sulfate, allantoin hydroxy aluminum, aluminum chloride, aluminum sulfate, zinc carbolate, tannic acid, citric acid, lactic acid, etc.), algefacient (menthol, camphor, etc.), hormone (estrogenic hormone, adrenal cortical hormone, etc.), antihistamine agent (diphenhydramine hydrochloride, chlorpheniramine maleate, glycyrrhizinic acid derivative, etc.), skin oil suppressing agent (estradiol, estrone, ethynylestradiol, etc.), corneum peeling and dissolving agent (sulfur, salicylic acid, resorcin, etc.), bactericidal agent (benzalkonium chloride, benzethonium chloride, halocarbane, 2,4,4-trichloro-2-hydroxyphenol, trichlorocarbanide, acetic acid tocopherol, zinc pyrithione, chlorhexidine, hinokitiol, phenol, isopropylmethylphenol, etc.), antipruritic agent (diphenhydramine hydrochloride, chlorpheniramine maleate, camphor, etc.) and the like.
   Examples of the whitening component include α-albutyne, hydroquinone, isoflavone, kojic acid, vitamin C (sodium ascorbate, magnesium ascorbyl phosphate, ascorbyl stearate, ascorbyl palmitate, ascorbyl dipalmitate, disodium ascorbyl sulfate, glucoside ascorbate, etc.), glutathione, calcium pantetheine sulfate, placental extract, ellagic acid, rucinol, mulberry leaf extract, peony root extract, sanguisorba officinalis root extract, Aesculus hippocastanum bark extract, polygonum bistorta extract, Sophora angustifolia extract, lonicera japonica extract, scutellaria baicalensis root extract, reynoutria japonica extract, chamomilla recutita extract, tranexamic acid, linoleic acid, linolenic acid, sulfur, wheat germextract, lactic acid, glycol acid, licorice extract and the like. Of these, α-arbutin, kojic acid, vitamin C (ascorbic acids), ellagic acid and rucinol are particularly preferable.
(2) preparation for topical application to the scalp
   When a preparation for topical application is to be applied to the scalp (hereinafter to be also referred to as "preparation for topical application to the scalp"), the preparation may contain, along with a blood circulation promoting component, a hair growing increasing component or hair growth-promoting component generally contained in hair tonic, hair growing agent, hair growth agent and the like.

Examples of the hair growing increasing component and hair growth-promoting component include nucleic acid (adenosine and phosphate ester thereof, etc.), vitamins (vitamin A, vitamin B1, vitamin B2, vitamin B6, dialkylmonoamine derivative, biotin, calcium pantothenate, panthenyl alcohol, etc.), amino acids (glutamine, methionine, cystine, cysteine, serine, leucine, tryptophan, etc.), mononitroguaiacol, glyceride pentadecanate, hinokitiol, coleus extract, chlorophyll, allantoin, estradiol, ethynylestradiol, hypericum perforatum, scutellaria baicalensis root, calendula officinalis flower, garlic extract, carrot extract, spironolactone, progesterone, cyproterone acetate, 4-androsten-3,17-dione, eugenyl glucoside, eugenia caryophyllus extract, quachalarata extract, duke extract, hops extract, minoxidil, t-flavanone and the like. Of these, nucleic acid, amino acids, glyceride pentadecanate and minoxidil are particularly preferable.

In the present invention, a preferable combination of a supplement and a preparation for topical application, and an effect expected to be afforded by the combination are as follows.
(1) Preferable combination for the prophylaxis or improvement of skin pigmentation
   The combination includes, but is not limited to:
   a combination of at least one kind selected from cysteine, cystine, glutathione and tocopherol acetate, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from vitamin C (sodium ascorbate, magnesium ascorbyl phosphate, ascorbyl stearate, ascorbyl palmitate, ascorbyl dipalmitate, disodium ascorbyl sulfate, glucoside ascorbate), and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from arbutin, kojic acid, ellagic acid, rucinol, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from mulberry leaf extract, peony root extract, sanguisorba officinalis root extract, Aesculus hippocastanum bark extract, polygonum bistorta extract, Sophora angustifolia extract, lonicera japonica extract, scutellaria baicalensis root extract, reynoutria japonica extract, chamomilla recutita extract, wheat germextract and licorice extract, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.), and the like.
(2) Preferable combination for improvement of moisturization
   The combination includes, but is not limited to, a combination of at least one kind selected from vitamin B2, vitamin B6, vitamin E, vitamin A, vitamin C and coenzyme Q10, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from collagen, hyaluronic acid and chondroitin sulfate, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from Minerals (zinc), and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from squalene (shark liver extract, olive oil, cottonseed oil, avocado oil, etc.), and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.), and the like.
(3) Preferable combination for hair growth or hair growing
   The combination includes, but is not limited to, a combination of at least one kind selected from histidine and glutamine, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from vitamins such as vitamin A, vitamin B2, vitamin B6, pantothenic acid (vitamin B5), vitamin C, vitamin E, vitamin F (unsaturated fatty acid), biotin (vitamin H), vitamin B15 (pangamic acid), vitamin Q (ubiquinone), coenzyme Q10, folic acid and the like, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from nucleic acid such as adenosine and phosphate ester thereof and the like, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from various minerals containing zinc, sulfur, iodine, iron, calcium, copper and the like, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.), and the like.
(4) Preferable combination for antiaging such as anti-wrinkles and the like
   The combination includes, but is not limited to, a combination of at least one kind selected from amino acid such as proline, alanine, glutamine, cystine, teanin and the like, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from vitamins such as vitamin B1 (thiamine), vitamin B2 (riboflavin), niacin (nicotinic acid), vitamin A, vitamin E, vitamin B15 (pangamic acid), vitamin C, vitamin Q (ubiquinone) and the like, and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from Polyphenol (anthocyanin, catechin, cacaomas polyphenol, quercetin, isoflavone, quercetin, chlorogenic acid), and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.),
   a combination of at least one kind selected from Carotenoid (β-carotene, lutein, lycopene), and a blood circulation improving agent (containing, for example, a blood circulation promoting component such as capsinoids, pyrrolidone carboxylic acid and salts thereof, esters of pyrrolidone carboxylic acid, arginine and salts thereof, arginine oligomer (octamer to dodecamer), vitamin P, etc.) and the like.

The input tool is not particularly limited as long as a user (one in need of information such as client, seller and the like) can input information of a supplement or a preparation for topical application. For example, a terminal having an input device such as touch panel, key board, mouse and the like, a mobile terminal (e.g., mobile information terminal, mobile-phone, etc.) and the like can be mentioned.

The output tool is not particularly limited as long as information such as a preparation for topical application and/or a supplement determined by a computer, a method of combined use thereof and the like can be output. For example, output devices such as various displays, printer and the like can be mentioned.

The input tool and output tool may be independent apparatuses, or one apparatus may have both an input device and an output device and function as an input tool and an output tool.

As the input tool and output tool, a mobile terminal can be used. Examples of the mobile terminal include, but are not limited to, mobile computer, PDA, mobile-phone and the like.

The format of the database is not particularly limited as long as it can store information of respective correlations, and may be any general database format.

The medium to store the database is not particularly limited, either, and memory media such as information memory medium (semiconductor memory, flexible disk, hard disk etc.), optical memory medium (CD, DVD etc.) and the like can be used, that are readable/writable by the computer.

The source of information provided by the present invention may be a client itself, or a mediator (e.g., product seller and the like).

The present invention is explained in more detail in the following by referring to the drawings.

The present invention also provides a method of providing, to a client, information of a combination of a supplement suitable for the client and a preparation for topical application (Fig. 1).

The method includes at least the steps S1 - S5 in Fig. 1.

S1 is a step of inputting a part desired by a client to be improved and an improvement effect desired for said part into an input tool.

S2 is a step for a computer to determine a composition of nutritional components suitable for the improvement effect desired for the part desired by the client to be improved, by referring to the information stored in a database 10, in which information of the correlation between improvement effects desired for parts to be improved and compositions of nutritional components is registered in advance, and extracting, from the information contained in the database 10, information of the composition of the nutritional components corresponding to the improvement effect desired for the part to be improved.

S3 is a step for the computer to determine a preparation for topical application suitable for delivering the determined nutritional components to the part to be improved, by referring to the information stored in a database 11, in which information of the correlation between parts of the body and preparations for topical application suitable for delivering nutritional components to the parts is registered in advance, and extracting, from the information contained in the database 11, a preparation for topical application suitable for the part desired by the client to be improved.

S4 is a step for the computer to determine a supplement containing the composition of the determined nutritional components, by referring to a database 12, in which supplements and compositions of nutritional components thereof is registered in advance, and extracting, from the information contained in the database 12, information of a supplement corresponding to the composition of the nutritional components determined in S2.

S5 is a step of outputting the information of the supplement and the preparation for topical application as determined by the computer in S1 - S4 into an output tool.

The information output in S5 may be that of a single supplement or a single preparation for topical application, or two or more kinds thereof when there are two or more kinds of supplements and preparations for topical application suitable for the improvement effect desired by the client.

In addition to S1 - S5, a step of outputting a method of using the determined supplement and preparation for topical application in combination and/or the effect expected by the combined use into an output tool (S6, not shown) may be included. S6 may be performed simultaneously with S5. Moreover, the information of the effect expected by the combined use may be stored in a database prepared separately, or preserved as additional information in the existing database such as database 11 and the like.

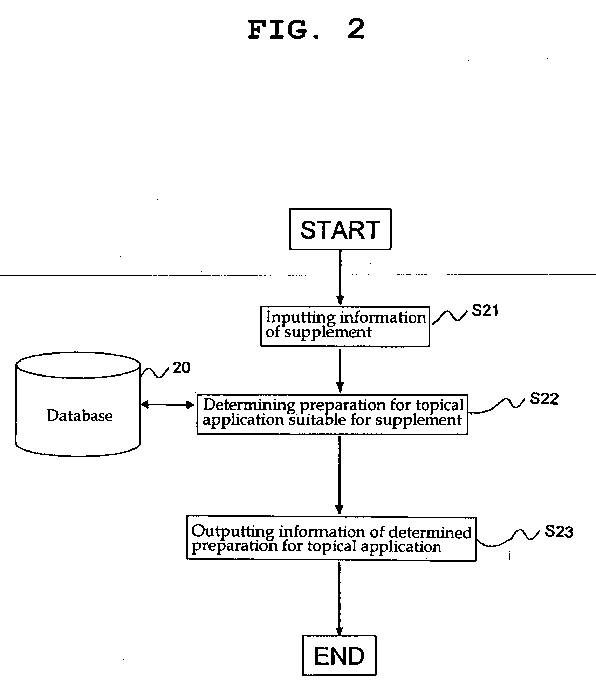

The present invention also provides a method of providing, to a client, information of a preparation for topical application suitable for the supplement selected by the client (Fig. 2).

The method includes at least the steps S21 - S23 in Fig. 2.

S21 is a step of inputting information (name of supplement and the like) of a supplement into an input tool.

S22 is a step for a computer to select a preparation for topical application suitable for delivering nutritional components contained in the supplement input in S21 to a part to be improved, by referring to the information stored in a database 20, in which information of the correlation between supplements and preparations for topical application suitable for delivering nutritional components contained in supplements to parts to be improved is registered in advance, and extracting, from the information contained in the database 20, information of the preparation for topical application suitable for delivering the nutritional components contained in the supplement (input in S21) to the part to be improved.

The correlation between a supplement and a preparation for topical application may be a direct correlation between the supplement and the preparation for topical application, or an indirect correlation via a composition of nutritional components contained in the supplement.

S23 is a step of outputting the information (name of the preparation for topical application and the like) of the preparation for topical application selected by the computer in S22 into an output tool.

The information output in S23 may be one kind of a preparation for topical application, or two or more kinds thereof.

In addition, a step of outputting a method of using the supplement and selected preparation for topical application in combination and/or the effect expected by the combined use into an output tool (S24, not shown) may be further included. S24 may be performed simultaneously with S23. Moreover, the information of the effect expected by the combined use may be stored in a database prepared separately, or preserved as additional information in database 20.

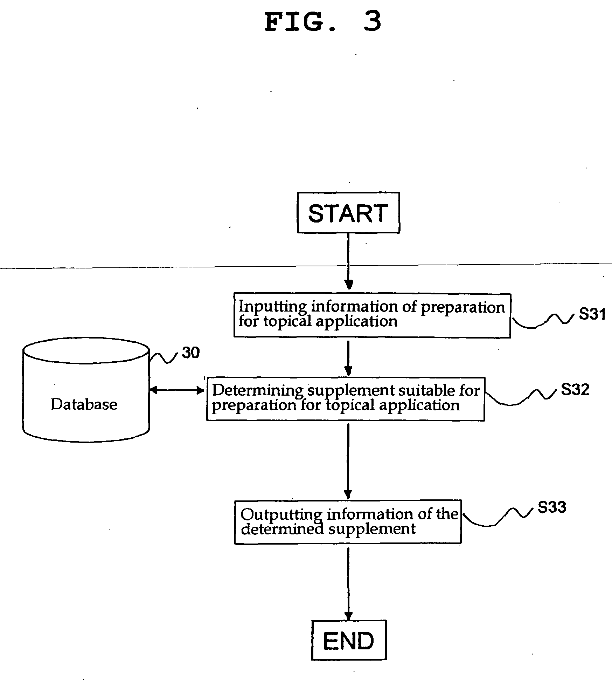

The present invention also provides a method of providing, to a client, information of a supplement suitable for a preparation for topical application selected by the client (Fig. 3).

The method includes at least the steps S31 - S33 in Fig. 3.

S31 is a step of inputting the information (name of the preparation for topical application and the like) of a preparation for topical application into an input tool.

S32 is a step for a computer to select a supplement suitable for delivering the preparation for topical application (input in S31) to an applied part, by referring to the information stored in a database 30, in which the information of the correlation between preparations for topical application and supplements suitable for delivering preparations for topical application to applied parts to be improved is registered in advance, and extracting, from the information contained in the database 30, information of a supplement suitable for delivering the preparation for topical application (input in S31) to a part to be improved.

S33 is a step of outputting the information (name of supplement and the like) of the supplement determined by the computer in S32 into an output tool.

The information output in S33 may be one kind of a preparation for topical application, or two or more kinds thereof.

In addition, a step of outputting a method of using the supplement and the preparation for topical application in combination and/or the effect expected by the combined use into an output tool (S34, not shown) may be further included. S34 may be performed simultaneously with S33. Moreover, the information of the effect expected by the combined use may be stored in a database prepared separately, or preserved as additional information in the database 30.

The present invention also provides a method for the prophylaxis or improvement of skin pigmentation, which comprises orally administering a supplement comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide before or after transdermal application of a preparation for topical application comprising at least one kind of blood circulation promoting component selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.

In the above-mentioned method, "before or after" means that the order of a transdermal application of a preparation for topical application and an oral application of a supplement may be any, and a suitable time may be present between the two applications as long as the above-mentioned supplement can more effectively afford a prophylactic or improvement effect for skin pigmentation by a transdermal application of a preparation for topical application as compared to the absence of the preparation for topical application. The time is, though not particularly limited, for example, 0 - 3 hr, preferably 30 min - 2 hr, more preferably about 30 min - 1 hr.

The present invention also provides a kit for the prophylaxis or improvement of skin pigmentation, comprising a preparation for topical application comprising at least one kind of blood circulation promoting component selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract, and a supplement comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, which is used for oral administration to a test subject to whom the preparation for topical application is applied. The kit may contain an instruction manual indicating an optimal method of using the kit for the prophylaxis or improvement of skin pigmentation.

The present invention also provides an agent for the prophylaxis or improvement of skin pigmentation, comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, which is used for the prophylaxis or improvement of skin pigmentation of a test subject to whom a preparation for topical application comprising at least one kind of blood circulation promoting component selected from the group consisting of capsinoids, capsinoid derivative, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract is applied to a part where prophylaxis or improvement of the skin pigmentation is desired.

The present invention further provides a system for providing, to a client, information relating to a combination of a supplement and a preparation for topical application suitable for the client. The system can be used for practicing the method explained above by referring to Fig. 1.

The system comprises
an input tool for inputting a part desired by a client to be improved,
a first database 10 containing registered information of the correlation between improvement effects desired for parts to be improved and compositions of nutritional components,
a second database 11 containing registered information of the correlation between parts of the body and preparations for topical application suitable for delivering nutritional components to the parts,
a third database 12 wherein supplements and compositions of nutritional components thereof are registered, and
an output tool indicating the information of a supplement and a preparation for topical application determined by a computer.

The computer (central processing unit) integrating the system refers to the first database 10 and determines the composition of nutritional components suitable for the part to be improved (corresponding to S2 in Fig. 1), refers to the second database 11 and determines a preparation for topical application suitable for delivering the composition of nutritional components suitable for the part to be improved to said part (corresponding to S3 in Fig. 1), and refers to the third database 12 and determines a supplement containing the determined composition of nutritional components (corresponding to S4 in Fig. 1).

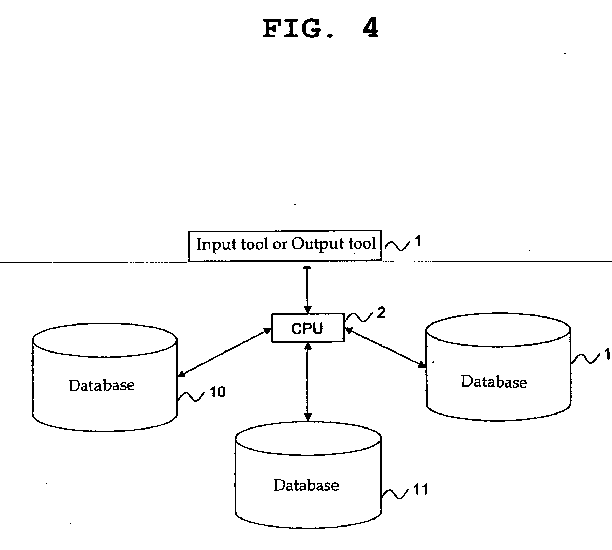

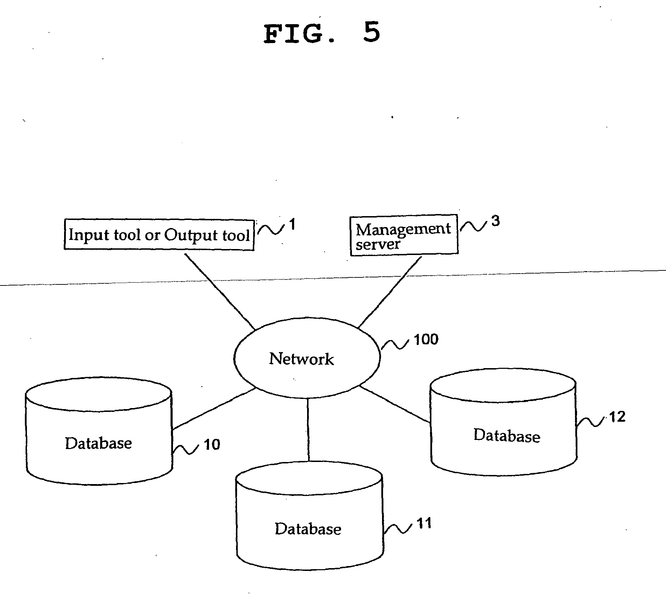

As shown in Fig. 4, this system may be integrated by a local central processing unit (CPU) 2 or, as shown in Fig. 5, integrated via a network 100 by a CPU installed in an external management server 3. The network 100 is not particularly limited as long as it can deliver each input and output information, and program information. Examples thereof include computer network, such as LAN, WAN (e.g., internet and the like), wireless communication network and the like. As shown in Fig. 4 and Fig. 5, an input tool may also serve as an output tool, or they may be independently prepared. While Fig. 4 and Fig. 5 each show embodiment of only one of the systems of the present invention, it is clear to those of ordinary skill in the art that an input tool, an output tool, CPU and respective databases may be locally connected or connected to a network, which is the same for the systems of other embodiments to be described below.

Moreover, since the method and system of the present invention are suitable for use in mail-order sales since they can be practiced through a network as mentioned above.

While one example of the mail-order sales is described in the following, the present invention is not limited to this embodiment alone.

A user desirous of purchasing an article accesses an online shop of a seller via an information terminal (including a mobile information terminal) connected to the internet, an exclusive line and the like.

According to the instructions indicated by the seller on an output tool (e.g., display) of an information terminal, the user inputs necessary information by operations using a key board, a mouse, a button and the like.

By the method and system of the present invention, information for the user is provided on the output tool.

Moreover, a merchandise purchase button, a detail display button and the like displayed in the existing online shopping sites and the like may be displayed along with the information.

The present invention also provides a system for providing information of a preparation for topical application suitable for a supplement selected by a client. The system can be used for practicing the method explained above by reference to Fig. 2.

The system comprises
an input tool for inputting information of a supplement,
a database 20 containing registered information of the correlation between supplements and preparations for topical application, and
an indication tool for indicating a preparation for topical application determined by a computer.

A computer (CPU) integrating the system refers to the database 20 and determines a preparation for topical application suitable for the supplement (corresponding to S22 in Fig. 2).

The present invention also provides, to a client, a system for providing a supplement suitable for the preparation for topical application selected by the client. The system can be used for practicing the method explained above by reference to Fig. 3.

The system comprises
an input tool for inputting information of a preparation for topical application,
a database 30 containing registered information of the correlation between preparations for topical application and supplements, and
an indication tool for indicating a supplement determined by a computer.

A computer (CPU) integrating the system refers to the database 30 and selects a supplement suitable for a preparation for topical application (corresponding to S32 in Fig. 3).

The present invention also provides a program for allowing a computer to function as the aforementioned system. The program may be described in any language as long as the computer can function as the aforementioned system.

The memory medium for the program is, for example, a memory storage tool such as information memory media (semiconductor memory, flexible disk, hard disk etc.), optically readable media (CD-ROM, DVD etc.) and the like, that are readable/writable by the computer. In addition, the program may be stored in an external memory medium by connecting to a computer network (LAN, WAN such as internet and the like, internet wireless communication network etc.) that supplies program information as a carrier wave.

The present invention also provides a combination product of a supplement and a preparation for topical application, and further, a combination product including a combination information document of the supplement and the preparation for topical application.

As the preparation for topical application, those mentioned above can be used.

The combination information document includes a combined effect provided by the combined use (the effect here is a concept also encompassing an action) and a method of combined use.

The combined effect is a concept corresponding to the "improvement effects desired for parts to be improved" explained earlier.

The method of combined use includes, for example, conditions for combining a supplement and a preparation for topical application, such as instructions of the timing of administration of the two and the like.

Particularly preferable embodiments of the combination product in the present invention are as follows.

A combination product wherein a supplement contains at least one kind selected from the group consisting of cysteine, cysteine derivative, cystine, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, and a preparation for topical application contains a blood circulation promoting component, which contains information for combined use that the product is used for the prophylaxis or improvement of skin pigmentation.

A combination product wherein a supplement contains at least one kind selected from the group consisting of mineral, vitamin, amino acid, carbohydrates, lipid, inositol, sulfur, nucleic acid, collagen, sesaminol and citric acid, and a preparation for topical application contains a blood circulation promoting component, which contains a written matter describing information for combined use that the product is used for promoting hair growth in the scalp.

The present invention further provides a supplement including a written matter describing a combined used with a preparation for topical application. In this case, the above-mentioned information for combined use, particularly an effect of combined use and a method of combined use, is preferably indicated in addition to the "combined used with a preparation for topical application".

Particularly preferable embodiments of the supplement in the present invention are as follows.

A supplement wherein the supplement contains at least one kind selected from cysteine, cysteine derivative, cystine, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, and a preparation for topical application contains a blood circulation promoting component, which contains a written matter describing information for combined use that the supplement is used for the prophylaxis or improvement of skin pigmentation.

A supplement wherein the supplement contains at least one kind selected from mineral, vitamin, amino acid, carbohydrates, lipid, inositol, sulfur, nucleic acid, collagen, sesaminol and citric acid, and a preparation for topical application contains a blood circulation promoting component, which contains a written matter describing information for combined use that the supplement is used for promoting hair growth in the scalp.

Moreover, the present invention provides a preparation for topical application including a written matter describing a combined used with a supplement. In this case, the above-mentioned information for combined use, particularly an effect of combined use and a method of combined use, is preferably indicated in addition to the "combined used with a supplement".

A particularly preferable embodiment of the preparation for topical application in the present invention is as follows.

A preparation for topical application wherein a supplement contains at least one kind selected from the group consisting of cysteine, cysteine derivative, cystine, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, and a preparation for topical application contains a blood circulation promoting component, which contains a written matter describing information for combined use that the preparation is used for the prophylaxis or improvement of skin pigmentation.

A preparation for topical application wherein a supplement contains at least one kind selected from the group consisting of mineral, vitamin, amino acid, carbohydrates, lipid, inositol, sulfur, nucleic acid, collagen, sesaminol and citric acid, and a preparation for topical application contains a blood circulation promoting component, which contains a written matter describing information for combined use that the preparation is used for promoting hair growth in the scalp.

Examples are given below to demonstrate that the preparation for topical application and the supplement provided according to the method of the present invention can provide clients with merchandise that can achieve high client satisfaction, wherein an effect of combined use of the preparation for topical application and the supplement determined by the method is established.

### Examples

### Example 1 Increase in topical concentration of systemically administered component by a preparation for topical

### application

A hairless mouse (HR-1, female) was intraperitoneally injected with a trypan blue solution (0.4%, 300 µl). After injection, 100 µl of 5% capsiate (dissolved in an ethanol solution) was applied uniformly using a pipet chip to the right side of the back. As a control, 100 µl of ethanol alone was uniformly applied to the left side of the back by a similar method. After 15-20 minutes elapsed from application, skin pigmentation of a blue dye derived from the trypan blue solution was confirmed only on the capsiate application side. When 30 minutes elapsed from application, clear skin pigmentation (infiltration) of the blue dye was observed on the capsiate application site (see Fig. 6). When 5% capsiate (dissolved in an ethanol solution) was applied to the mouse auricle on the side opposite to the side of capsiate application on the back, pigmentation of a blue dye was observed also in the vicinity of the auricle after 30-60 minutes elapsed. This demonstrated that an application of capsiate having a vasodilating effect to the skin brings about a blood circulation promoting effect in the application site, and the substance (dye) administered into the abdomen is selectively collected to the application site.

### Example 2 Synergistic effect by oral administration of tocopherol acetate and transdermal application of blood circulation promoter

The synergistic effect of tocopherol acetate and dihydrocapsiate was confirmed using a pigmentation induction mouse based on the inhibition of pigmentation induced by ultraviolet beam (UVB).
(1) Before the start of ultraviolet beam (UVB) irradiation, tocopherol acetate (200 µl) was administered orally once a day for 3 days to a hairless mouse (12-week-old, female) using a gavage needle.
(2) After the 3-day oral administration in (1), the hairless mouse was irradiated with UVB (80-90 mJ/cm²).
(3) Thirty minutes after the irradiation in (2), tocopherol acetate (200 µl) was administered orally to the hairless mouse using a gavage needle. Five minutes after the oral administration, 28 µl of 5 wt% dihydrocapsiate dissolved in 50 wt% ethyl alcohol was applied to the hairless mouse on the left side of the back.

Treatments (2) and (3) were performed 3 times a week (Monday, Wednesday and Friday) and treatment (3) alone was performed twice a week (Tuesday and Thursday). The skin on the back of the mouse after about 3 weeks from the start of the UV irradiation was visually evaluated. It was confirmed that the left side of the back applied with dihydrocapsiate showed less pigmentation as compared to the non-application site (right side of the back) (Fig. 8a). In a control experiment using a hairless mouse applied with a solvent (50 wt% ethyl alcohol) instead of dihydrocapsiate, a clear difference was not observed between the right side and the left side of the back (Fig. 8b). In another control experiment using a hairless mouse applied with dihydrocapsiate alone and free of oral administration of tocopherol acetate, UVB irradiation induced pigmentation in the application site (Fig. 8c). Moreover, it was also confirmed that the pigmentation suppressive effect confirmed in this experiment on the left side on the back of the hairless mouse (dihydrocapsiate application site) disappears and induction of pigmentation begins when the administration of tocopherol acetate and the application of dihydrocapsiate are ceased and UVB is continuously irradiated according the method of (2).

These results reveal that both the single oral administration of tocopherol acetate (200 µl) and the single application of dihydrocapsiate failed to suppress induction of pigmentation by UVB irradiation onto the back of the hairless mouse. However, it was confirmed that the synergistic effect provided by the oral administration of tocopherol acetate and the transdermal application of dihydrocapsiate can suppress induction of pigmentation by UVB, which could not be suppressed when each of these methods was applied singly.

### Example 3 Synergistic effect of oral administration of cystine and transdermal application of blood circulation promoter

An experiment similar to that in Example 2 was performed using cystine as an oral agent. In particular, 50 mg of cystine was dissolved in 150 µl of a 0.5% aqueous CMC (carboxymethylcellulose) solution and used as an oral administration sample, and 5% dihydrocapsiate (50% aqueous ethanol solution) was used as a dermal application sample. Ultraviolet beam (UVB) was irradiated without preliminary administration, and the synergistic effect afforded by the oral administration agent and the blood circulation promoter on the blackness suppressive effect on the pigmentation of the back of a mouse was confirmed according to the experiment schedule similar to that in Example 2. Suppression of pigmentation induction in the dihydrocapsiate application group was visually evaluated. The levels of pigmentation after about 3 weeks and about 4 weeks from the start of the UV irradiation are shown in Fig. 9. It was confirmed that pigmentation induction was clearly suppressed in the dihydrocapsiate application group as compared to the control (50% aqueous ethanol solution application group).

### Visual evaluation criteria

0: No pigmentation
1: Spots of pale pigmentation found in a part
2: Spots of pale pigmentation found on the entire back
3: Spots of pigmentation found on the entire back (partly deep pigmentation was found)
4: Spots of deep pigmentation found on the entire back
5: Deep pigmentation found on the entire back

### Reference Example 1

The vasodilating effects (blood circulation improving effect) of capsiate and capsaicin were compared using auricles of a hairless mouse.

### [Test method]

A sample was applied to the right ear of a hairless mouse (HR-1, female) and a control was applied to the left ear thereof, and the development of a red spot was visually evaluated.

### Samples:

(1) Capsiate (1 wt% or 5 wt% dissolved in liquid paraffin)
(2) Capsaicin (1 wt% or 5 wt% dispersed in liquid paraffin

### Control:

Liquid paraffin

### [Results]

The results of the visual evaluation reveal that the presence of a red spot at 1 wt% capsaicin, no development of a red spot with capsiate, and a clear red spot on auricles from 30 minutes onward after the application of 5 wt% each of capsaicin and capsiate. Moreover, the developed red spot completely disappeared in about 2 hr for capsiate whereas the red spot maintained the initial state even after 2 hr for capsaicin even at 1 wt%. On the other hand, no red spot was observed by the application of the control liquid paraffin. Note that capsaicin did not dissolve completely in liquid paraffin, and the evaluation was performed in a dispersion state for both 1 wt% and 5 wt%.

It was clarified from the results that capsaicin shows a strong blood circulation promoting action and long sustainability, whereas capsiate shows mild sustainability and intensity and is a more preferable blood circulation promoting component for cosmetic uses.

Formulation examples are given below wherein capsinoids are used as blood circulation improving agents in preparations for topical application. Other blood circulation improving agents can be used in a similar manner in place of capsinoids.

### [Formulation Example 1] cream

| | |
|---|---|
| capsiate | 1.0 wt% |
| stearic acid | 2.0 |
| polyoxyethylene(25)cetyl ether | 3.0 |
| glyceryl monostearate | 2.0 |
| octyldodecanol | 10.0 |
| cetyl alcohol | 6.0 |
| reduction lanolin | 4.0 |
| squalane | 9.0 |
| 1,3-butyleneglycol | 6.0 |
| polyethylene glycol (1500) | 4.0 |
| preservative | q.s. |
| flavor | q.s. |
| antioxidant | q.s. |
| purified water | q.s. |

### [Formulation Example 2] milk lotion

| | |
|---|---|
| capsiate | 2.0 wt% |
| sorbitan sesquioleate | 2.0 |
| polyoxyethyleneoleoylether | 2.5 |
| stearyl alcohol | 0.5 |
| hydrogenated palm oil | 3.0 |
| liquid paraffin | 35.0 |
| dipropylene glycol | 6.0 |
| polyethylene glycol (400) | 4.0 |
| carboxyvinyl polymer (1% aqueous solution) | 15.0 |
| preservative | q.s. |
| flavor | q.s. |
| purified water | balance |
| [Formulation Example 3] gel capsiate | 0.5 wt% |
| liquid paraffin | 12.0 |
| tri(2-ethylhexane acid) glyceryl | 50.0 |
| sorbit | 10.0 |
| polyethylene glycol (400) | 5.0 |
| acylmethyltaurine | 5.0 |
| polyoxyethylene(20)isocetyl ether | 10.0 |
| flavor | q.s. |
| preservative | q.s. |
| purified water | balance |

### [Formulation Example 4] essence

| | |
|---|---|
| dihydrocapsiate | 1.0 wt% |
| p-methoxycinnamic acid-2-ethylhexyl | 4.0 |
| 3,3'-(1,4-phenylenedimethylidyne) bis(7,7-dimethyl-2-oxo-bicyclo(2,2,1)heptane-1-methane sulfonic acid) | 4.0 |
| polyoxyethylenecetyl ether | 2.0 |
| glycerol monostearate | 2.0 |
| stearic acid | 3.0 |
| cetyl alcohol | 1.0 |
| lanolin | 3.0 |
| liquid paraffin | 5.0 |
| 2-ethylhexyl stearate | 3.0 |
| 1,3-butyleneglycol | 6.0 |
| flavor | q.s. |
| preservative | q.s. |
| purified water | balance |

### [Formulation Example 5] lotion

| | |
|---|---|
| capsiate | 3.0 wt% |
| glycerol | 3.0 |
| sorbitol | 2.0 |
| polyoxyethylene(20)oleyl ether | 1.0 |
| ethanol | 15.0 |
| zinc paraphenol sulfonate | 0.2 |
| flavor | q.s. |
| preservative | q.s. |
| purified water | balance |

### [Formulation Example 6] hair growth agent

| | |
|---|---|
| capsiate | 2.0 wt% |
| Swertia japonica extract | 0.1 |
| hyaluronic acid | 0.2 |
| hinokitiol | 0.2 |
| vitamin B6 | 0.5 |
| vitamin E | 0.5 |
| urea | 2.5 |
| propylene glycol | 4.0 |
| ethanol | 50.0 |
| flavor | q.s. |
| preservative | q.s. |
| purified water | balance |

### [Formulation Example 7] peel off jel pack

| | |
|---|---|
| capsiate | 2.0 wt% |
| jojoba oil | 1.0 |
| squalane | 1.0 |
| PEG 400 | 5.0 |
| sorbitol | 5.0 |
| ethanol | 8.0 |
| polyvinyl alcohol | 10.0 |
| polyvinyl acetate emulsion | 15.0 |
| titanium oxide | 5.0 |
| talc | 10.0 |
| POE sorbitan monostearic acid ester | 1.0 |
| flavor | q.s. |
| preservative | q.s. |
| purified water | balance |

### Industrial Applicability

According to the method of the present invention, the information of a supplement and a preparation for topical application, which are suitable for the object of a client, can be obtained based on a report of the client as to the part desired by the client to be improved and an improvement effect desired for the part. Consequently, the consumer appetite of the client can be encouraged and merchandise that earns high level of client satisfaction can be provided to the client.

Moreover, using the combination agent, supplement and preparation for topical application of the present invention, the effect of the supplement can be exhibited more effectively.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2005-219669 filed in Japan, the contents of which are incorporated in full herein by this reference.

## Claims

1. A method of providing information of a supplement and a preparation for topical application, which are suitable for a client, which comprises
a step of inputting a part desired by the client to be improved and an improvement effect desired for said part into an input tool,
a step for a computer to determine a composition of nutritional components suitable for the improvement effect desired for the part desired by the client to be improved, by referring to the information stored in a database, in which information of the correlation between improvement effects desired for parts to be improved and compositions of nutritional components is registered in advance, and extracting, from the information contained in the database, information of the composition of the nutritional components suitable for the improvement effect desired for the part to be improved,
a step for the computer to determine a preparation for topical application, which is suitable for delivering the determined nutritional components to the part to be improved, by referring to the information stored in a database, in which information of the correlation between parts of the body and preparations for topical application suitable for delivering nutritional components to the parts is registered in advance, and extracting, from the information contained in the database, a preparation for topical application suitable for the part desired by the client to be improved,
a step for the computer to determine a supplement containing the composition of the determined nutritional components, by referring to a database, in which supplements and compositions of nutritional components thereof is registered in advance, and extracting, from the information in the database, information of a supplement corresponding to the composition of the determined nutritional components, and
a step of outputting the information of the supplement and the preparation for topical application as determined by the computer to an output tool.

2. The method of claim 1, further comprising a step of outputting a method of using the determined supplement and the preparation for topical application in combination and/or the effect expected by the combined use to the output tool.

3. The method of claim 1 or 2, wherein the part desired by the client to be improved is the skin.

4. The method of any one of claims 1 to 3, wherein the improvement effect desired for the part to be improved is at least one kind selected from the group consisting of improvement and/or prophylaxis of skin wrinkles, improvement and/or prophylaxis of skin pigmentation, promotion of hair growth in the scalp and prevention of gray hair.

5. The method of any one of claims 1 to 4, wherein the composition of the determined nutritional components comprises at least one kind selected from the group consisting of vitamin, amino acid, peptide, protein, mineral, dietary fiber, fatty acid, carbohydrate, nucleic acid, organic acid, fungi, extract from plants and animals, and lipid.

6. The method of any one of claims 1 to 5, wherein the determined preparation for topical application is a blood circulation improving agent comprising a blood circulation promoting component.

7. The method of claim 6, wherein the blood circulation promoting component comprises at least one kind of active ingredient selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.

8. The method of any one of claims 1 to 7, wherein the determined supplement comprises at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, the determined preparation for topical application comprises a blood circulation promoting component, and the improvement effect desired for the part to be improved is an improvement and/or prophylaxis of skin pigmentation.

9. The method of any one of claims 1 to 7, wherein the determined supplement comprises at least one kind selected from the group consisting of mineral, vitamin, amino acid, carbohydrates, lipid, inositol, sulfur, nucleic acid, collagen, sesaminol and citric acid, the preparation for topical application comprises a blood circulation promoting component, and the improvement effect desired for the part to be improved is promotion of hair growth in the scalp.

10. A method of providing information of a preparation for topical application suitable for a supplement selected by a client, which comprises
a step of inputting information of the supplement into an input tool,
a step for a computer to determine a preparation for topical application suitable for delivering nutritional components contained in the supplement to a part to be improved, by referring to the information stored in a database, in which information of the correlation between supplements and preparations for topical application suitable for delivering nutritional components contained in the supplements to parts to be improved is registered in advance, and extracting, from the information contained in the database, information of the preparation for topical application suitable for delivering the nutritional components contained in the input supplement to the part to be improved, and
a step of outputting the information of the preparation for topical application as determined by the computer into an output tool.

11. The method of claim 10, further comprising a step of outputting a method of using the supplement and the determined preparation for topical application in combination and/or the effect expected by the combined use to the output tool.

12. The method of claim 10 or 11, wherein the determined preparation for topical application is a blood circulation improving agent containing a blood circulation promoting component.

13. The method of claim 12, wherein the blood circulation promoting component comprises at least one kind of active ingredient selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.

14. A method of providing information of a supplement suitable for a preparation for topical application selected by a client, which comprises
a step of inputting information of the preparation for topical application into an input tool,
a step for a computer to select a supplement suitable for delivering the preparation for topical application to a part to be improved, by referring to the information stored in a database, in which information of the correlation between preparations for topical application and supplements suitable for delivering preparations for topical application to parts to be applied is registered in advance, and extracting, from the information contained in the database, information of a supplement suitable for delivering the preparation for topical application to the part to be applied, and
a step of outputting the information of the supplement as determined by the computer into an output tool.

15. The method of claim 14, further comprising a step of outputting a method of using the supplement and the determined preparation for topical application in combination and/or an effect expected by the combined use to the output tool.

16. The method of claim 14 or 15, wherein the determined preparation for topical application is a blood circulation improving agent comprising a blood circulation promoting component.

17. The method of claim 16, wherein the blood circulation promoting component comprises at least one kind of active ingredient selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.

18. A method for the prophylaxis or improvement of skin pigmentation, which comprises orally administering a supplement comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide before or after transdermal application of a preparation for topical application comprising at least one kind of blood circulation promoting component selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract.

19. A kit for the prophylaxis or improvement of skin pigmentation, consisting of a preparation for topical application comprising at least one kind of blood circulation promoting component selected from the group consisting of capsinoids, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract, and a supplement comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, which is used to be orally administered to a test subject to whom the preparation for topical application is applied.

20. An agent for the prophylaxis or improvement of skin pigmentation, comprising at least one kind selected from the group consisting of cysteine, cysteine derivative, cystine, cystine derivative, vitamin C, polyphenol, tannic acid, vitamin E, vitamin E derivative, catechin, niacin, hydroxytyrosol, unsaturated fatty acid, vitamin P, vitamin Q, glutathione, isoflavone, guava, selenium and zinc oxide, which is used for the prophylaxis or improvement of skin pigmentation of a test subject to whom a preparation for topical application comprising at least one kind of blood circulation promoting component selected from the group consisting of capsinoids, capsinoid derivative, pyrrolidone carboxylic acid and salts thereof, esters of pyrrolidone carboxylic acid, arginine and salts thereof, arginine oligomer, nicotinic acid, niacin, capsaicin, vitamin E, Swertia japonica extract, cepharanthine, isoleucine, carpronium chloride, minoxidil, Ginger Tincture, Cantharides Tincture, benzyl nicotinate, γ-oryzanol, garlic extract, carrot extract, angelicae radix extract, Gentiana lutea extract, raspberry ketone, astaxanthin, saponin, vitamin B1, flavonoid, rutin, allyl sulfide, niacinamide, tocopherol nicotinate, camphor, aminobutyric acid, cepharanthine, Cnidium officinale, placental extract, nasturtium officinale extract, Daucus carota extract, Sophora angustifolia extract, wheat germ extract, saffron crocus extract, zingiber officinale root extract, Cnidium officinale extract, Paeonia suffruticosa extract, Angelica keiskei extract, Fennel extract, Chamolilla extract, edible burdock extract, Crataegus extract, shiitake mushroom extract, calamus root extract, Crataegus oxyacantha extract, juniper extract, Thymus vulgaris extract, Aurantii Nobilis Pericarpium extract, Persicae Semen extract, bitter orange extract, Rosa multiflora extract, grape leaf extract, Aesculus hippocastanum extract, Melissa officinalis extract, Citrus junos extract, Coicis semen extract, rosemary extract and Ginkgo extract is applied to a part where prophylaxis or improvement of the skin pigmentation is desired.
